# EUROPEAN PATENT SPECIFICATION

(11) **EP 4 226 964 B1**
(45) Date of publication and mention of the grant of the patent: **22.04.2026**
(21) Application number: 20957600.8
(22) Date of filing: 12.10.2020
(51) Int. Cl.: A61M 25/09

(54) **MEDICAL DEVICE**
MEDIZINISCHE VORRICHTUNG
DISPOSITIF MÉDICAL

(43) Date of publication of application: 16.08.2023
(73) Proprietor: ASAHI INTECC CO., LTD., Seto-shi, Aichi 489-0071 (JP)
(72) Inventor: ISHIKAWA, Masatomo, Seto-shi, Aichi 489-0071 (JP); OSHIMA, Fumiyoshi, Seto-shi, Aichi 489-0071 (JP)
(74) Representative: Winter, Brandl - Partnerschaft mbB
(86) International application number: PCT/JP2020/038525
(87) International publication number: WO 2022/079771

(56) References cited:
- WO-A1-2017/210018
- JP-A- 2005 534 407
- JP-A- 2013 103 075
- JP-A- 2017 136 158
- JP-A- 2019 520 128
- JP-A- 2019 521 742
- JP-A- 2019 521 742
- JP-A- H06 197 980
- JP-A- H10 272 118
- JP-A- H10 272 118
- JP-A- S62 261 371
- US-A1- 2007 016 131
- US-A1- 2007 032 746
- US-A1- 2007 032 746
- US-A1- 2012 227 457

## Description

### TECHNICAL FIELD

The present disclosure relates to a medical device.

### BACKGROUND ART

Medical devices such as guide wires and catheters are used for minimally invasive treatment or examination inside body lumens such as the circulatory system and the digestive system. In such medical devices, it is preferable to be able to detect the position of the medical device once inserted inside the body. For example, JP 2008-99713 A discloses a configuration in which a plurality of magnetic pieces are arranged in a medical tube to detect the position of the medical tube. Furthermore, JP 2006-280591 A discloses a configuration in which a source coil serving as a magnetic field generating element is arranged on the insertion portion of a medical device inserted inside the body, and a hollow organ inside which the insertion portion has been inserted is detected by detecting the magnetic field generated by the source coil. Moreover, JP 2019-520129 W discloses a configuration of a magnetic mechanism provided in a medical device, in which the position of the medical device is detected by detecting the magnetic mechanism (magnetic region), which includes a material such as a ferromagnet or paramagnet that is magnetized by an externally applied magnetic field. In addition, JP 2013-103075 A discloses a configuration in which a magnet is embedded in the distal end portion of a medical device such as a guide wire.

JP 2019 521742 A discloses a medical device comprising a catheter adapter subassembly and a wire subassembly including a wire, wherein one of the catheter adapter subassembly and the wire subassembly includes a permanent magnet element. WO 2017 210018 A1 describes a medical device comprising an elongate shaft having a diameter, an outer surface, a distal tip, and a proximal end, the diameter of the elongate shaft sized to be inserted within an intravenous catheter, at least a portion of the elongate shaft having a first magnetic region and a discontinuity in the first magnetic region providing a diameter transition such that the shaft includes an increased diameter region.

### SUMMARY OF INVENTION

### TECHNICAL PROBLEM

However, a configuration in which a magnet is arranged in the medical device as in JP 2008-99713 A and JP 2013-103075 A, and a configuration in which a coil is arranged in the medical device as an electromagnet as in JP 2006-280591 A can be difficult to adopt because of reasons including the effect on the properties of the medical device such as the flexibility, and the increased complexity and size of the structure of the medical device. Furthermore, a configuration that provides a magnetic region that is magnetized by an externally applied magnetic field as in JP 2019-520129 W can be difficult to adopt due to reasons including the need for a device to apply the external magnetic field when detecting the position of the medical device, which causes an increase in the complexity and size of the configuration of the position detection system as a whole. Therefore, a technique has been sought that detects the position of a medical device and suppresses the effects on the performance of the medical device, while also suppressing an increase in the complexity and size of the device.

### SOLUTION TO PROBLEM

This object is solved by means of a medical device according to the invention as defined in claim 1. Further aspects are disclosed in the dependent claims. The present disclosure can be implemented as the aspects described below.
(1) According to an aspect of the present disclosure, a medical device is formed in an elongated outer shape, and has a distal end side inserted inside a body when in use. The medical device includes a magnetized member in which a magnetized portion containing magnetized martensitic stainless steel is formed in a portion of the distal end side. The medical device is further provided with a high coercive force layer formed so as to cover at least a portion of a surface of the magnetized member, and contains a material with a higher coercive force than martensitic stainless steel, wherein the magnetized portion has the high coercive force layer magnetized together with the magnetized member. With this configuration, because the magnetized portion has the high coercive force layer magnetized together with the magnetized member, the magnetic field strength of the magnetized portion can be increased compared to a case where the high coercive force layer is not provided. As a result, the accuracy with which the position of the medical device is detected using the magnetized portion can be increased. In addition or as an alternative, a pair of permanent magnets having lines of magnetic force extending in the same direction as the magnetized portion is provided on both sides of the magnetized portion. With this configuration, a decrease in the magnetic force of the magnetized portion can be suppressed.
   According to a medical device of the present aspect, because a magnetized member having a magnetized portion containing magnetized martensitic stainless steel is formed in a portion of the distal end side, a magnetized portion that can be used to detect the position of the medical device can be provided by magnetizing the magnetized member, which contains martensitic stainless steel. Therefore, unlike a case where a member that is magnetized, such as a permanent magnet, is additionally incorporated into the medical device, the position of the distal end portion of the medical device can be detected while also suppressing an increase in the complexity and size of the configuration of the medical device. Furthermore, unlike a case where a member that is magnetized, such as a permanent magnet, is additionally incorporated inside the medical device, a decrease in the strength of the medical device associated with formation of the magnetized portion can be suppressed, and the risk of the magnetized portion becoming detached from the medical device can be suppressed. In addition, because the magnetized portion containing magnetized martensitic stainless steel is provided, it is not necessary to apply an additional external magnetic field when detecting the position of the medical device. As a result, it is possible to suppress an increase in the complexity and size of the configuration of the overall system, which includes the medical device and a position detection device of the medical device.
(2) In the medical device of the above aspect, the magnetized member may be a core shaft, and the magnetized portion may be provided in a distal end portion of the core shaft. With this configuration, by forming the magnetized portion by magnetizing the core shaft included in the medical device, it is possible to detect the position of the distal end portion of the core shaft.
(3) In the medical device of the above aspect, a plurality of the magnetized portions may be provided spaced apart from each other in an axial direction of the medical device. With this configuration, by detecting the plurality of magnetized portions such that the positions are distinguished, the three-dimensional movement of the distal end portion of the medical device can be more accurately recognized.
(4) In the medical device of the above aspect, at least two magnetized portions among the plurality of magnetized portions may have lines of magnetic force extending in different directions from each other. With this configuration, by detecting the plurality of magnetized portions such that the directions in which the lines of magnetic force extend are distinguished, the accuracy with which the three-dimensional movement of the distal end portion of the medical device is recognized can be further increased.
(5) In the medical device of the above aspect, a distal end portion of the medical device may be provided with a bent portion at which the medical device is bent in a specific direction, a first magnetized portion, being one of the plurality of magnetized portions, may be provided further toward a distal end side than the bent portion, and a second magnetized portion, being another one of the plurality of magnetized portions, may be provided further toward a proximal end side than the bent portion. With this configuration, by detecting the first magnetized portion, which is further toward the distal end side than the bent portion, and the second magnetized portion, which is further toward the proximal end side than the bent portion, such that the positions and the directions in which the lines of magnetic force extend are distinguished, the accuracy with which the three-dimensional movement of the distal end portion of the medical device is recognized can be increased. For example, when a load is applied to the distal end of the medical device and a deformation such as bending occurs in the medical device, the deformation mainly occurs further on the distal end side than the bent portion. Therefore, by using the position of second magnetized portion, which is provided toward the proximal end side from the bent portion, and the direction the lines of magnetic force extend as a reference, even in a case where a load is applied to the distal end of the medical device, the detection accuracy of the three-dimensional movement of the location in which the first magnetized portion is provided can be increased.
(6) In the medical device of the above aspect, the first magnetized portion and the second magnetized portion may have lines of magnetic force extending in different directions from each other. With this configuration, by detecting the first magnetized portion and the second magnetized portion such that the directions in which the lines of magnetic force extend are distinguished, the accuracy with which the three-dimensional movement of the distal end portion of the medical device is recognized can be further increased.

The present disclosure can be realized as various aspects other than those described above, and can be realized as aspects such as a production method of a medical device, and a system provided with the medical device that also detects the position of the medical device after being inserted inside the body.

### BRIEF DESCRIPTION OF DRAWINGS

Fig. 1 is a partial sectional view showing a configuration of the distal end portion of a guide wire of a first embodiment (not covered by the subject-matter of the claims).
Fig. 2 is a flowchart showing a production method of a core shaft.
Fig. 3A is an explanatory diagram showing a magnetizing process that magnetizes the distal end portion of the core shaft.
Fig. 3B is an explanatory diagram showing the magnetized portion formed by the magnetizing process in Fig. 3A.
Fig. 4A is an explanatory diagram showing the magnetizing process that magnetizes the distal end portion of a core shaft.
Fig. 4B is an explanatory diagram showing the magnetized portion formed by the magnetizing process in Fig. 4A.
Fig. 5 is a partial sectional view showing a configuration of the distal end portion of a guide wire of a second embodiment (not covered by the subject-matter of the claims).
Fig. 6 is a partial sectional view showing a configuration of the distal end portion of a guide wire of a third embodiment.
Fig. 7 is a flowchart showing a production method of a core shaft.
Fig. 8 is a partial sectional view showing a configuration of the distal end portion of a guide wire of a modification of the third embodiment.
Fig. 9 is a partial sectional view showing a configuration of the distal end portion of a guide wire of a fourth embodiment.
Fig. 10 is an explanatory diagram showing a measurement of the change in magnetic flux density in the axial direction when a magnetized portion has a magnetization direction parallel to the axial direction.
Fig. 11 is an explanatory diagram showing a measurement of the change in magnetic flux density in the axial direction when a magnetized portion has a magnetization direction perpendicular to the axial direction.
Fig. 12 is a partial sectional view showing a configuration of the distal end portion of a guide wire of a fifth embodiment.
Fig. 13 is a partial sectional view showing a configuration of the distal end portion of a guide wire of a sixth embodiment.

### EMBODIMENTS OF THE DISCLOSURE

### A. First Embodiment (not covered by the subject-matter of the claims):

### (A-1) Overall Configuration of Guide Wire:

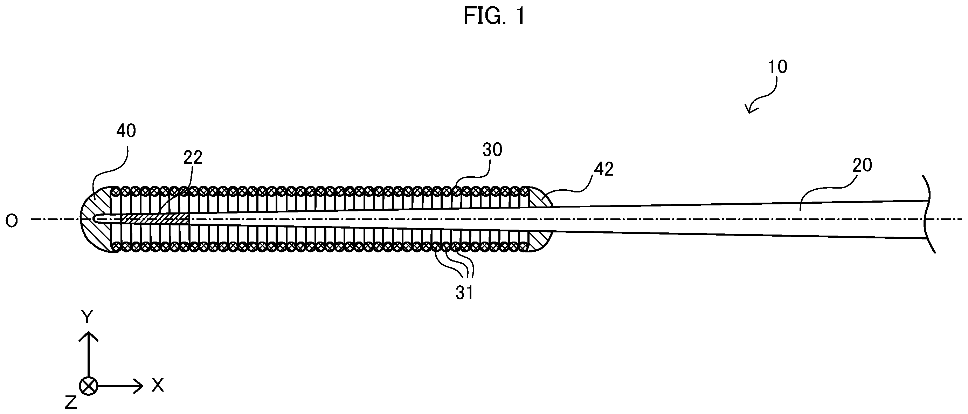

Fig. 1 is a partial sectional view showing a schematic configuration of a guide wire 10 of a first embodiment (not covered by the subject-matter of the claims). The guide wire 10 is a medical device which is formed in an elongated outer shape and used by being inserted from the distal end into the body, and is used, for example, when a catheter is inserted into a blood vessel or the digestive tract. The guide wire 10 includes a core shaft 20, an outer coil 30, a distal end joint portion 40, and a proximal end joint portion 42. In Fig. 1, the axis passing through the center of the guide wire 10 is indicated by an axis O (dot and dash line). In the present embodiment, the axis passing through the center of the core shaft 20 and the axis passing through the center of the outer coil 30 both coincide with the axis O. However, at least one of the axes passing through the center of the core shaft 20 and the outer coil 30 may be different from the axis O. The direction the axis O extends is also referred to simply as the "axial direction". Note that Fig. 1 schematically represents the arrangement of each portion, and does not exactly represent the dimensional ratios of each portion.

Fig. 1, and Fig. 3A to Fig. 7, Fig. 8, Fig. 12, and Fig. 13 described below illustrate mutually orthogonal X, Y, and Z axes. The X-axis corresponds to the axis direction of the guide wire 10, the Y-axis corresponds to the height direction of the guide wire 10, and the Z-axis corresponds to the width direction of the guide wire 10. The left side in Fig. 1 (negative X-axis direction) is referred to as the "distal end side" of the guide wire 10 and each component member, and the right side in Fig. 1 (positive X-axis direction) is referred to as the "proximal end side" of the guide wire 10 and each component member. Furthermore, of the two ends of the guide wire 10 and each component member in the axial direction (X-axis direction), the end located on the distal end side is referred to as the "distal end", and the other end located on the proximal end side is referred to as the "proximal end". In addition, the end portion including the distal end is referred to as the "distal end portion", and the end portion including the proximal end is referred to as the "proximal end portion". The guide wire 10 is inserted from the distal end side into the body, and is operated from the proximal end portion by an operator such as a physician. Fig. 1 shows a section of the guide wire 10 that includes the distal end portion.

The core shaft 20 is a member formed in an elongated outer shape that extends along the axis O, and is arranged from the distal end portion to the proximal end portion of the guide wire 10. Although the core shaft 20 of the present embodiment has a circular shape overall in a transverse cross-section perpendicular to the axis O, it may at least in part have a different shape, such as an ellipse-shaped cross-section. The distal end portion of the core shaft 20 has a tapered shape having a thicker diameter on the proximal end side, and a thinner diameter on the distal end side. Fig. 1 shows a portion of the core shaft 20 that has a decreasing diameter toward the distal end. At the distal end portion of the core shaft 20, the rate at which the diameter decreases toward the distal end of the core shaft 20 does not have to be constant. For example, as long as the core shaft 20 as a whole has a decreasing diameter toward the distal end, a portion that has a constant cross-sectional diameter may be provided in addition to a portion that has a decreasing diameter toward the distal end portion.

The core shaft 20 of the present embodiment is formed of stainless steel, and has an austenite phase and a martensite phase as phases with different crystal structures. Furthermore, as shown in Fig. 1, the core shaft 20 is provided with a magnetized portion 22 in a portion on the distal end side. The magnetized portion 22 contains magnetized martensitic stainless steel, and is formed by magnetizing a portion of the core shaft 20. The core shaft 20 is also referred to as a "magnetized member". The magnetization of the core shaft 20 and the magnetized portion 22 will be described in detail below.

The outer coil 30 is arranged so that the core shaft 20 penetrates through the interior, and is a substantially cylindrical member formed by spirally winding a wire 31. As a result of providing the outer coil 30, the flexibility and torquability of the distal end portion of the guide wire 10 are enhanced.

In the present embodiment, although the outer coil 30 is a single-thread coil formed by winding a single wire 31, it may also be a multi-thread coil formed by winding a plurality of wires 31 in a multi-thread pattern, a single-thread strand coil formed by winding a single strand formed by twisting together a plurality of wires 31, or a multi-thread strand coil formed using a plurality of strands formed by twisting together a plurality of wires 31 and winding the strands in a multi-thread pattern. Furthermore, the outer coil 30 may be configured by an arbitrary combination of single-thread coils, multi-thread coils, single-thread strand coils, and multi-thread strand coils. The wire diameter of the wire 31 and the mean coil diameter of the outer coil 30 (mean outer diameter and mean inner diameter of the outer coil 30) can be arbitrarily set.

The wire 31 can be formed of a stainless alloy such as SUS304 and SUS316, a superelastic alloy such as nickel-titanium alloy, piano wire, or a radiolucent alloy such as nickel-chromium alloy and cobalt alloy. Furthermore, it may also be formed of gold, platinum, tungsten, tantalum, iridium, palladium, or a radiopaque alloy such as an alloy containing these elements (for example, platinum-nickel alloy). Note that the wire 31 may be formed of a known material other than those described above.

The distal end of the outer coil 30 is fixed to the distal end of the core shaft 20 by the distal end joint portion 40. On the other hand, the proximal end of the outer coil 30 is fixed to the core shaft 20 by the proximal end joint portion 42. The distal end joint portion 40 and the proximal end joint portion 42 may be formed of metal solder such as Ag-Sn alloy, Au-Sn alloy, Sn-Pb alloy, or Pb-Ag alloy. The distal end joint portion 40 and the proximal end joint portion 42 may be formed of the same material, or may be formed of different materials. Note that, in order to fix the core shaft 20 and the outer coil 30, the guide wire 10 may be further provided with a fixing portion other than the distal end joint portion 40 and the proximal end joint portion 42.

### (A-2) Magnetization of Core Shaft and Magnetized Portion:

Hereinafter, a phase change in the stainless steel of the core shaft 20 and the formation of the magnetized portion 22 upon magnetization of the core shaft 20 will be described.

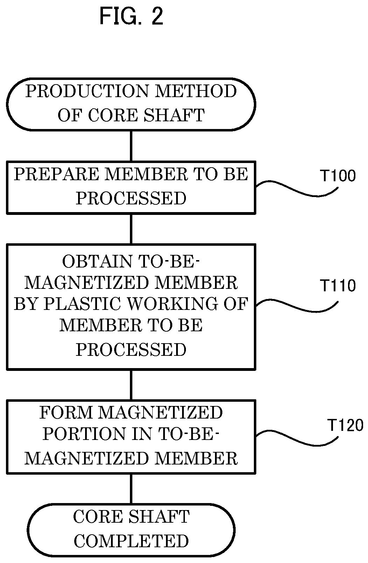

Fig. 2 is a flowchart showing a production method of the core shaft 20. When producing the core shaft 20, a member to be processed into the core shaft 20 is firstly prepared (step T100). In the present embodiment, the member to be processed is a rod-shaped (solid) or tubular (hollow) member with a constant cross-sectional diameter, and a member composed of austenitic stainless steel (such as SUS304 or SUS316), which is paramagnetic, is prepared.

Next, the member to be processed is subjected to plastic working such that the diameter of the member to be processed decreases toward the distal end portion, and as a result a to-be-magnetized member is obtained (step T110). Examples of plastic forming include centerless grinding, drawing, and swaging. By subjecting the member to be processed composed of austenitic stainless steel to the plastic working described above, in the portion having a relatively high processing intensity, or more specifically, in the portion near the distal end of the core shaft 20 in which the extent of diameter reduction is relatively large, the austenite crystal undergoes a plastic-induced transformation to form a martensite crystal. That is, a processing-induced martensitic transformation occurs, and at least a portion of the austenite phase forms the martensite phase, which is a ferromagnetic material. Therefore, the to-be-magnetized member obtained by plastic working has the martensite phase near the distal end. The crystal structure of the stainless steel constituting the core shaft 20 and the to-be-magnetized member can be identified by an X-ray diffraction measurement.

Then, as a result of applying an external magnetic field and magnetizing at least a portion of the part near the distal end of the to-be-magnetized member that has been subjected to plastic working, the magnetized portion 22 is formed (step T120), which completes the core shaft 20. The magnetizing device that generates the external magnetic field mentioned above may be a device capable of generating a magnetic field that can form the magnetized portion 22, and includes, for example, a permanent magnet, or an air core coil that generates a magnetic field by applying an electric current.

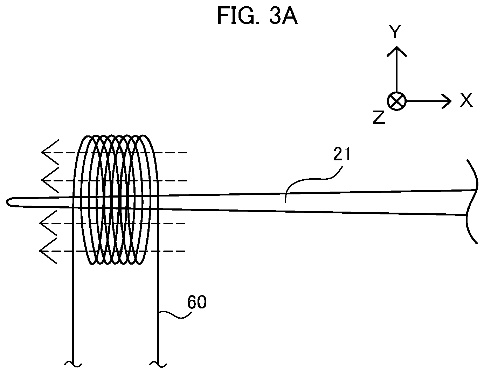

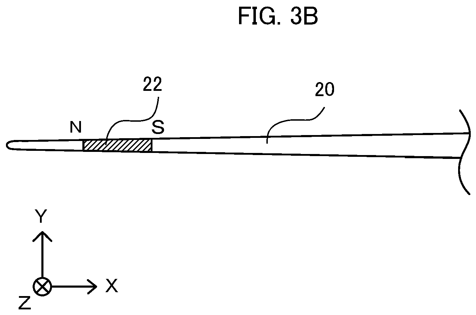

Fig. 3A is an explanatory diagram showing the magnetizing process that magnetizes the distal end portion of the to-be-magnetized member 21 using a magnetizing device provided with an air core coil 60. Furthermore, Fig. 3B is an explanatory diagram showing the magnetized portion 22 formed by the magnetizing process shown in Fig. 3A. In Fig. 3A, the location of the to-be-magnetized member 21 in which the magnetized portion 22 is to be formed is magnetized by being arranged inside the air core coil 60. In Fig. 3A, as an example, the direction of the magnetic field inside the air core coil 60 is in the negative X-axis direction parallel to the axis O, and is indicated by a dashed arrows.

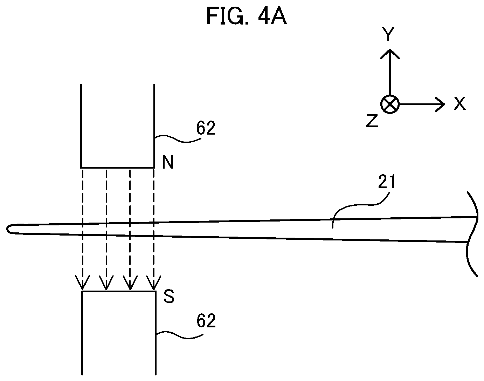

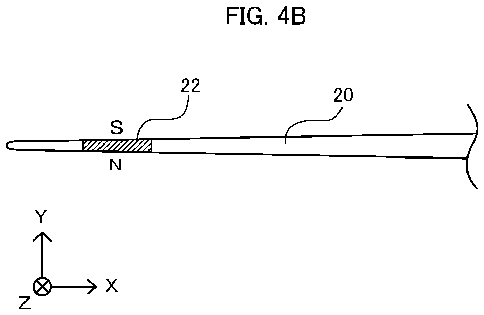

Fig. 4A is an explanatory diagram showing the magnetizing process that magnetizes the distal end portion of the to-be-magnetized member 21 using a magnetizing device provided with permanent magnets 62. Furthermore, Fig. 4B is an explanatory diagram showing the magnetized portion 22 formed by the magnetizing process shown in Fig. 4A. In Fig. 4A, the permanent magnets 62 are arranged and magnetized so that they face each other via the location of the to-be-magnetized member 21 in which the magnetized portion 22 is to be formed, which is placed between them. In Fig. 4A, as an example, the direction of the magnetic field generated by the magnetizing device is in the negative Y-axis direction perpendicular to the axis O, and is indicated by a dashed arrows.

As shown in Fig. 3B and Fig. 4B, the magnetized portion 22 is formed in a magnetization direction corresponding to the direction of the applied magnetic field. The magnetization direction may be different from Fig. 3A or Fig. 4B. By changing the direction of the magnetic field generated by the magnetizing device, the magnetization direction of the magnetized portion 22 can be arbitrarily adjusted. As the strength of the external magnetic field applied by the magnetizing device is increased, the strength of the magnetization of the magnetized portion 22 increases. A stronger magnetization of the magnetized portion 22 is preferable, and it is most desirable for the magnetization of the magnetized portion 22 to be in a saturated magnetization state.

As the stainless steel that constitutes the member to be processed into the core shaft 20, when stainless steel that readily undergoes a martensitic transformation due to processing is used, it becomes easier to form a magnetized portion 22 having a stronger magnetization. Among austenitic stainless steels, for example, SUS304 undergoes a martensitic transformation due to processing more readily than SUS316, and SUS302 even more readily undergoes a martensitic transformation. Furthermore, as the stainless steel that readily undergoes a martensitic transformation, a stainless steel other than austenitic stainless steel may be used. Examples of stainless steel other than austenitic stainless steel that readily undergoes a martensitic transformation include SUS444 and SUS434, which are ferritic stainless steels, and SUS630, which is a precipitation hardening stainless steel.

After the core shaft 20 is prepared as described above, the distal end portion of the core shaft 20 is inserted into the outer coil 30, and the distal end of the core shaft 20 and the distal end of the outer coil 30 are brazed together to form the distal end joint portion 40. Furthermore, the proximal end of the outer coil 30 and the core shaft 20 are brazed together to form the proximal end joint portion 42. Consequently, the guide wire 10 is obtained.

As a result of inserting the guide wire 10 provided with the magnetized portion 22 in the distal end portion as described above into the body, and detecting the strength and direction of the magnetic field inside the body, it is possible to detect the position of the distal end portion of the guide wire 10 inside the body. In order to detect how far the guide wire 10 has advanced inside the body, it is preferable for the position of the magnetized portion 22 to be as close as possible to the distal end of the guide wire 10.

A known magnetic sensor capable of detecting the strength and direction of the magnetic field generated by the magnetized portion 22 can be used to detect the position of the magnetized portion 22. Examples of the magnetic sensor include a GSR sensor (GHz-Spin-Rotation Sensor) sensor, a magnetoresistive effect device (MR), a magnetic impedance device (MI), and a superconducting quantum interference device (SUQUID). In order to detect the position of the magnetized portion 22, for example, a magnetic sensor array in which a plurality of such magnetic sensors are vertically and horizontally arranged in a matrix pattern may be used. The magnetic sensor array is, for example, arranged on a table on which a human body, which is subjected to a procedure using the guide wire 10, is lying horizontally. Alternatively, the magnetic sensor array may be configured to be worn by a human body which is subjected to a procedure using the guide wire 10. When the magnetic sensor array is worn by a human body, it may be configured having a band shape and be wrapped around the human body, or configured as a garment or hat. In these cases, the magnetic sensors may be arranged along the shape of the human body. Furthermore, plate-shaped magnetic sensor arrays may be three-dimensionally arranged on each of at least one of the front and rear sides of the human body, and on at least one of the left and right sides of the human body.

According to the guide wire 10 of the present embodiment configured as described above, the magnetized portion 22 is formed by magnetizing the core shaft 20 that is originally included in the guide wire 10. Therefore, unlike a case where a member that is magnetized, such as a permanent magnet, is additionally incorporated into the guide wire, the position of the distal end portion of the guide wire 10 can be detected while also suppressing an increase in the complexity and size of the configuration of the guide wire 10.

Further, according to the present embodiment, because the magnetized portion 22 is formed by magnetizing the core shaft 20, for example, unlike a case where the magnetized portion is formed by additionally incorporating a member composed of a permanent magnet or the like into the guide wire, a decrease in the strength of the guide wire 10 accompanying the formation of the magnetized portion 22 can be suppressed. Moreover, unlike a case where the magnetized portion is formed by additionally incorporating a member composed of a permanent magnet or the like into the guide wire, it is possible to reduce the risk of detachment of the magnetized portion that has been formed from the guide wire 10.

In addition, according to the present embodiment, because the magnetized portion 22 containing magnetized martensitic stainless steel is provided, it is not necessary to apply an additional external magnetic field when detecting the position of the guide wire 10. As a result, it is possible to suppress an increase in the complexity and size of the configuration of the overall system, which includes the guide wire 10 and a position detection device of the guide wire 10.

In the present embodiment, the magnetizing process that forms the magnetized portion 22 by magnetizing the to-be-magnetized member 21 is performed prior to the assembly process of the guide wire 10, but other configurations are possible. For example, after performing the assembly process, or more specifically, after brazing the outer coil 30 to the to-be-magnetized member 21 not yet provided with the magnetized portion 22, and forming the distal end joint portion 40 and the proximal end joint portion 42, it is possible to apply an external magnetic field to the core shaft 20 after the outer coil 30 has been joined. At this time, if the outer coil 30 is a member composed of a material other than a ferromagnetic material, the outer coil 30 is not magnetized, and only the core shaft 20 is magnetized. Note that the outer coil 30 can also be magnetized when the outer coil 30 contains a ferromagnetic material, and such a configuration is described later as a fifth embodiment.

### B. Second Embodiment (not covered by the subject-matter of the claims):

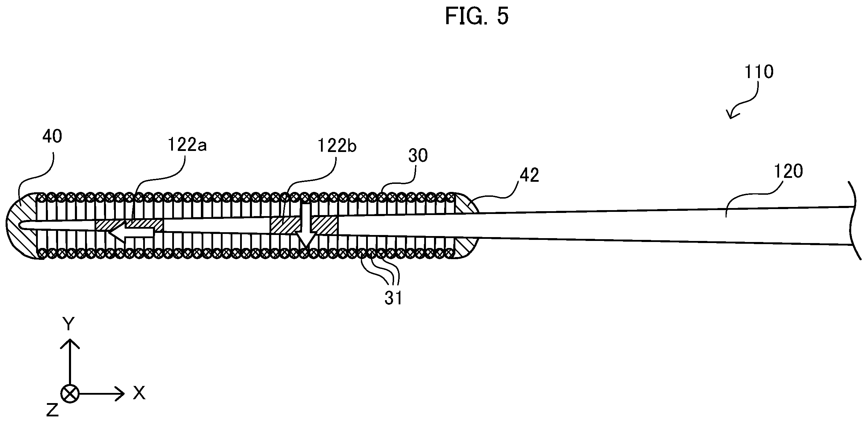

Fig. 5 is a partial sectional view showing, in the same manner as Fig. 1, a schematic configuration of the distal end portion of a guide wire 110 of a second embodiment (not covered by the subject-matter of the claims). In the second embodiment (not covered by the subject-matter of the claims), those parts that are common with the guide wire 10 of the first embodiment (not covered by the subject-matter of the claims) are given the same reference numerals. The guide wire 110 differs from the guide wire 10 in that it includes a core shaft 120 formed having a plurality (two in Fig. 5) of magnetized portions 122a and 122b instead of the magnetized portion 22.

In the guide wire 110, the magnetized portion 122a and the magnetized portion 122b are provided separated from each other in the axial direction of the guide wire 110. Further, in the second embodiment (not covered by the subject-matter of the claims), the magnetized portion 122a and the magnetized portion 122b have different magnetization directions.

The magnetized portion 122a and the magnetized portion 122b can be formed by applying, with respect to specific locations of the to-be-magnetized member 21, or more specifically, the locations in which the magnetized portion 122a and the magnetized portion 122b is to be formed, external magnetic fields in different directions from each other. The application of the external magnetic fields may be performed, for example, in the same manner as the methods described in Fig. 3A and Fig. 4A. Specifically, for example, the magnetized portion 122a can be formed by the method shown in Fig. 3A, and the magnetized portion 122b can be formed by the method shown in Fig. 4A. As a result, magnetized portions are formed that generate lines of magnetic force extending in the same direction as the direction of the applied external magnetic field, and the magnetized portion 122a and the magnetized portion 122b are formed that generate lines of magnetic force extending in different directions from each other. In Fig. 5, the direction the lines of magnetic force extend for each of the magnetized portions 122a and 122b, that is, the directions of the external magnetic fields applied with respect to the to-be-magnetized member 21, are indicated by white arrows. As an example, Fig. 5 shows that the direction the lines of magnetic force of the magnetized portion 122a extend is in the negative X direction (axial direction), and that the direction the lines of magnetic force of the magnetized portion 122b extend is in the negative Y direction. Note that, as described in the first embodiment (not covered by the subject-matter of the claims), the magnetizing process for forming the magnetized portion 122a and the magnetized portion 122b may be performed prior to the assembly process of the guide wire 110, or may also be performed after the assembly process.

With this configuration, because the magnetized portions 122a and 122b are formed by magnetizing the core shaft 120 that is originally included in the guide wire 110, the same effects as in the first embodiment (not covered by the subject-matter of the claims) can be obtained. Further, in the core shaft 120, because the plurality of magnetized portions 122a and 122b are provided separated from each other in the axial direction, by distinguishing the positions when detecting the magnetized portions 122a and 122b, it becomes possible to more accurately recognize the three-dimensional movement of the distal end portion of the guide wire 110 (such as the position of the distal end of the guide wire 110 and the orientation and angle of the distal end portion of the guide wire 110). The distance between the magnetized portion 122a and the magnetized portion 122b may be appropriately set according to the sensitivity of the magnetic sensor, such that the magnetized portion 122a and the magnetized portion 122b can be distinguished.

In addition, according to the second embodiment (not covered by the subject-matter of the claims), the directions the lines of magnetic force of the magnetized portion 122a and the magnetized portion 122b extend are different from each other. Consequently, by detecting the magnetized portion 122a and the magnetized portion 122b such that the directions in which the lines of magnetic force extend are distinguished, the accuracy with which the three-dimensional movement of the distal end portion of the guide wire 110 is recognized can be further increased. In the present embodiment, the directions in which the lines of magnetic force of the magnetized portion 122a and the magnetized portion 122b extend are different, but the directions in which the lines of magnetic force extend may also be the same. Even in this case, by providing the plurality of magnetized portions that are separated from each other in the axial direction, compared to a case where a single magnetized portion is provided, the accuracy with which the three-dimensional movement of the distal end portion of the guide wire 110 can be recognized can be increased.

The magnetization directions of the magnetized portion 122a and the magnetized portion 122b may be different from those in Fig. 5. Furthermore, when the magnetization direction of the magnetized portion 122a and the magnetization direction of the magnetized portion 122b are different from each other, the magnetization direction of the magnetized portion 122a and the magnetization direction of the magnetized portion 122b do not have to be orthogonal to each other. However, in order to accurately distinguish between the magnetized portion 122a and the magnetized portion 122b, the angle between the direction the lines of magnetic force of the magnetized portion 122a extend and the direction the lines of magnetic force of the magnetized portion 122b extend is preferably 20° or more, and more preferably 45° or more.

For example, when the magnetization directions of the magnetized portion 122a and the magnetized portion 122b are parallel to the axial direction and are in the same direction, the magnetized portion 122a and the magnetized portion 122b may be magnetically coupled such that the boundary becomes blurred. In contrast, when the magnetization directions of the magnetized portion 122a and the magnetized portion 122b are parallel to the axial direction and are in opposite directions, the magnetic forces between the magnetized portions 122a and 122b cancel each other out, which may lead to attenuation of the magnetic forces, and as a result, accelerated deterioration of the performance relating to the detection of the magnetized portions. Furthermore, when the magnetization directions of the magnetized portion 122a and the magnetized portion 122b are perpendicular to the axial direction and are in the same direction, magnetic repulsion occurs between the magnetized portion 122a and the magnetized portion 122b, and the guide wire 110 may become twisted such that the operability of the guide wire 110 is reduced. In contrast, when the magnetization directions of the magnetized portion 122a and the magnetized portion 122b are perpendicular to the axial direction and are in opposite directions, the magnetized portion 122a and the magnetized portion 122b form a magnetic circuit, and the quantity of the lines of magnetic force reaching the magnetic sensor becomes attenuated, which may cause a decrease in the accuracy when specifying the position using the magnetic sensor. In order to suppress such inconveniences and increase the accuracy with which the three-dimensional movement of the distal end portion of the guide wire 110 is detected, the angle between the direction the lines of magnetic force of the magnetized portion 122a extend and the direction the lines of magnetic force of the magnetized portion 122b is preferably as close to 90° as possible.

In the second embodiment (not covered by the subject-matter of the claims), regardless of whether the magnetization directions of the magnetized portion 122a and the magnetized portion 122b are the same, it is preferable that the magnetization direction of the magnetized portion 122a provided on the distal end side is parallel to the axial direction, or as close to parallel as possible. Because the core shaft 120 has a shape having a decreasing diameter toward the distal end, by bringing the magnetization direction of the magnetized portion 122a, which is formed on the distal end side where the core shaft 120 has a relatively thin diameter, closer to the axial direction, it becomes easier to ensure a distance between the N pole and S pole of the magnetized portion 122a. As a result, it becomes easier to form a magnetized portion 122a that generates a stronger magnetic field.

In the second embodiment (not covered by the subject-matter of the claims), two magnetized portions 122a and 122b are provided, but three or more magnetized portions may be provided. Similarly, in this case, it is preferable that the lines of magnetic force of at least two magnetized portions among the three or more magnetized portions extend in different directions from each other in order to increase the accuracy with which the three-dimensional movement of the distal end portion of the guide wire 110 is recognized.

### C. Third Embodiment:

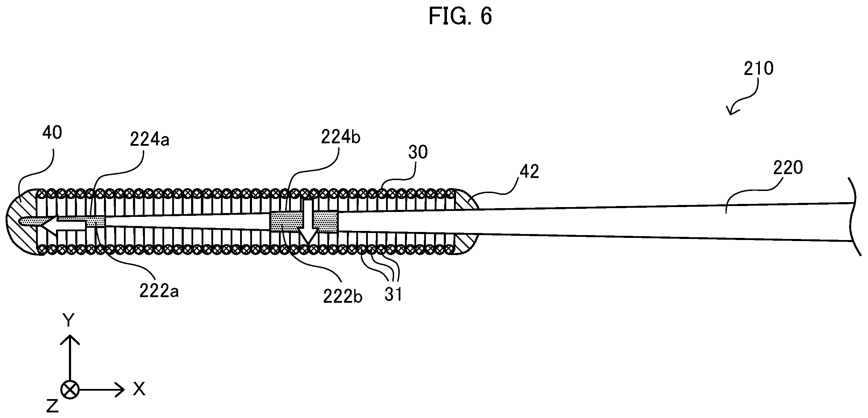

Fig. 6 is a partial sectional view showing, in the same manner as Fig. 1, a schematic configuration of the distal end portion of a guide wire 210 of a third embodiment. In the third embodiment, those parts that are common with the guide wire 10 of the first embodiment (not covered by the subject-matter of the claims) are given the same reference numerals. The guide wire 210 differs from the guide wire 10 in that it includes a core shaft 220 formed having plated portions 224a and 224b instead of the core shaft 20. In the guide wire 210, magnetized portions 222a and 222b are formed in regions in which the core shaft 220 and the plated portions 224a and 224b overlap.

The plated portions 224a and 224b are plated layers that cover specific locations that represent at least a portion of the surface of the core shaft 220, are provided separated from each other in the axial direction, and are formed of a magnetic material, that is, a ferromagnetic metal. The magnetic material constituting the plated portions 224a and 224b is preferably a magnetic material such as a platinum magnet in consideration of the stability and the like when the guide wire 210 is inserted into the body. Specifically, the magnetic material such as a platinum magnet is an alloy containing platinum as the main component, and iron (Fe), niobium (Nb), cobalt (Co), or the like. However, it is also possible to form the plated portions 224a and 224b using ferromagnetic metals other than the platinum alloy described above. The plated portions 224a and 224b include a material having a higher coercive force (intrinsic coercive force) HCJ than martensitic stainless steel. The plated portions 224a and 224b may have a higher coercive force HCJ than the core shaft 220, and preferably have a higher coercive force HCJ than martensitic stainless steel. The plated portions 224a and 224b are also referred to as "high coercive force layers". The magnetized portions 222a and 222b are each formed by magnetizing the core shaft 220 together with a portion of each of the plated portions 224a and 224b.

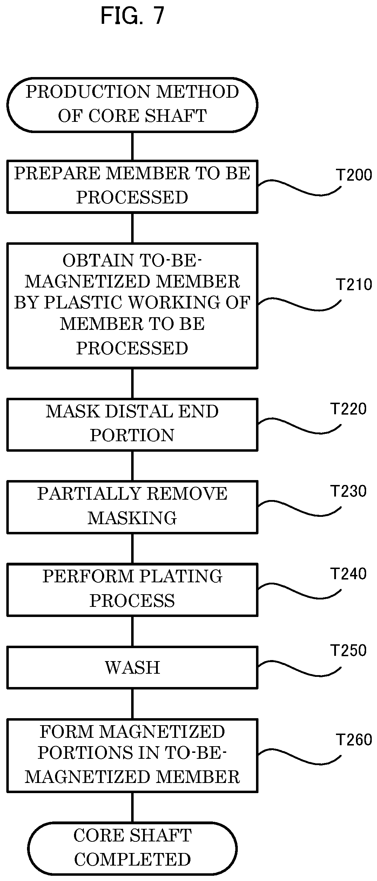

Fig. 7 is a flowchart showing a production method of the core shaft 220. When producing the core shaft 220, a member to be processed into the core shaft 20 is prepared (step T200), and the to-be-magnetized member 21 is obtained by subjecting the prepared member to be processed to plastic working (step T210). Step T200 and step T210 are the same processes as step T100 and step T110 in Fig. 2.

Next, the distal end portion of the to-be-magnetized member 21 that has been subjected to plastic working is masked (step T220). In step T220, masking is performed with respect to the to-be-magnetized member 21 so as to cover a wider area than the area of the to-be-magnetized member 21 which is immersed in the plating bath in the plating process described below. Then, a portion of the masking formed in step T220 is removed (step T230). In step T230, the masking provided on the regions of the to-be-magnetized member 21 in which the plated portions 224a and 224b are to be formed is removed.

Then, the plating process is performed (step T240). In the present embodiment, a film of the platinum alloy described above is formed by electroplating, but the plating process may also be performed using electroless plating. A suitable plating bath may be selected that enables a film of the platinum alloy described above to be formed. As a result of performing the plating process by immersing the to-be-magnetized member 21 in the plating bath in step T240, the plated portions 224a and 224b are formed in the regions where a portion of the masking was removed in step T230. After forming plated portions 224a and 224b, the to-be-magnetized member 21 is washed (step T250) to remove the plating solution adhering to the to-be-magnetized member 21, and to also remove the masking on the to-be-magnetized member 21.

Then, external magnetic fields are applied to the parts of the to-be-magnetized member 21 on which the plated portions 224a and 224b have been formed, which forms the magnetized portions 222a and 222b in regions overlapping the plated portions 224a and 224b (step T260), and the core shaft 220 is completed. Step T260 is a process in which external magnetic fields are applied in the same manner as step T120 in Fig. 2. In the regions where the external magnetic fields are applied, the core shaft 220, which contains martensitic stainless steel, and the plated portions 224a and 224b become magnetized to form the magnetized portions 222a and 222b. The application of the external magnetic fields can be performed, for example, in the same manner as the methods described in Fig. 3A and Fig. 4A. In Fig. 6, the directions the lines of magnetic force extend for each of the magnetized portions 222a and 222b, that is, the directions of the external magnetic fields applied with respect to the to-be-magnetized member 21 for forming the magnetized portions 222a and 222b, are indicated by white arrows. As an example, Fig. 6 shows that the direction the lines of magnetic force of the magnetized portion 222a extend is in the negative X direction (axial direction), and that the direction the lines of magnetic force of the magnetized portion 222b extend is in the negative Y direction. However, the magnetization directions of the magnetized portion 222a and the magnetized portion 222b may be different from those in Fig. 6. Here, the magnetization directions of the magnetized portion 222a and the magnetized portion 222b may be the same or different. Note that, as described in the first embodiment (not covered by the subject-matter of the claims), the magnetizing process (step T260) for forming the magnetized portions 222a and 222b may be performed prior to the assembly process of the guide wire 210, or may also be performed after the assembly process.

In the magnetizing process of step T260, when the parts of the to-be-magnetized member 21 on which the plated portions have been formed are arranged inside the air core coil 60 in the same manner as Fig. 3A, the axial direction lengths of the plated portions 224a and 224b are preferably longer than the axial direction length of the air core coil 60. For example, the plated portions 224a and 224b may each be about 1 mm longer than the air core coil 60 in the axial direction, that is, in the positive X direction and the negative X direction. As a result, the spread of magnetic flux in the magnetized portions formed in the magnetizing process can be suppressed. However, the axial direction length of the plated portions 224a and 224b can be made shorter than the axial direction length of the air core coil 60. In this case, portions of the to-be-magnetized member 21 that are not covered with the plated portions 224a and 224b are also magnetized and constitute the magnetized portions.

With this configuration, because the magnetized portions 222a and 222b are formed by magnetizing the core shaft 220 that is originally included in the guide wire 210, the same effects as in the first embodiment (not covered by the subject-matter of the claims) can be obtained. Furthermore, in the core shaft 220, because the plurality of magnetized portions 222a and 222b are provided separated from each other in the axial direction, the same effects as in the second embodiment (not covered by the subject-matter of the claims) are obtained. In addition, because the plated portions 224a and 224b composed of a magnetic material are provided on the surface of the core shaft 220, and the magnetized portions 222a and 222b are formed by applying external magnetic fields to the parts on which the plated portions 224a and 224b have been formed, the magnetic field strength of the magnetized portions 222a and 222b can be increased compared to a case where the plated portions 224a and 224b are not provided. As a result, because the difference between the magnetic field strength of the magnetized portions 222a and 222b and the magnetic field strength around the magnetized portions 222a and 222b can be made large, the accuracy with which the position of the distal end portion of the guide wire 210 is detected can be increased.

Further, in the present embodiment, because the plated portions 224a and 224b, which are high coercive force layers, are formed as metallic plated layers, it becomes easier to suppress the thickness of the plated portions 224a and 224b, and an increase in the size of the core shaft 220 due to the formation of the high coercive force layers can be suppressed. Moreover, because the core shaft 220 can be prevented from becoming thicker due to the plated portions 224a and 224b, it is possible to prevent the rigidity of the core shaft 220 from increasing to an undesirable level. Specifically, in the present embodiment, as shown in Fig. 6, at least a portion of the plated portion 224a is covered with the distal end joint portion 40, and is included in the distal end joint portion 40. Consequently, the effect of suppressing an increase in the size of the core shaft 220 due to providing the plated portion 224a can be further enhanced. However, if the extent of the increase in size of the core shaft 220 due to the plated portion 224a is within an allowable range, the plated portion 224a may also not be included in the distal end joint portion 40. Alternatively, the proximal end portion of the plated portion 224b may be included in the proximal end joint portion 42 to suppress an increase in the size of the core shaft 220 due to the formation of the plated portion 224b.

In addition, if the increase in size and rigidity of the core shaft 220 due to the formation of the plated portions 224a and 224b is within an allowable range, the high coercive forces layer may be formed by a method other than plating, and be formed of a magnetic material other than a metal. For example, the high coercive force layers may be formed of a magnetic ceramic such as ferrite. The configuration in which the high coercive force layers are formed of a metal plating as in the present embodiment is preferable because it is relatively easy to the control dimensions and the physical properties.

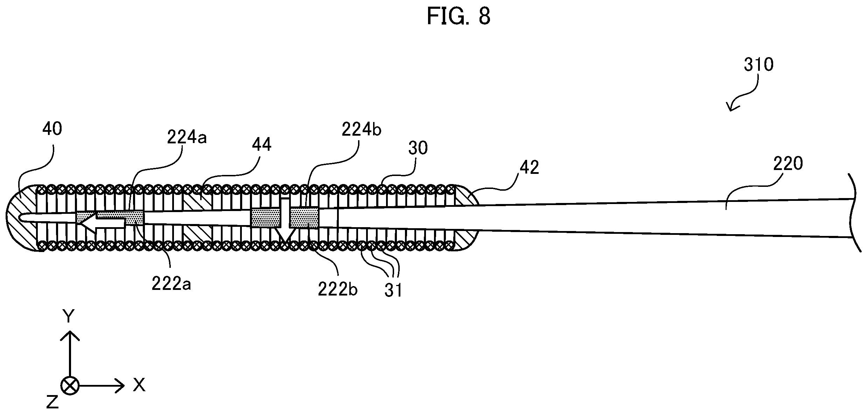

Fig. 8 is a partial sectional view showing, in the same manner as Fig. 1, a schematic configuration of the distal end portion of a guide wire 310 as a modification of the third embodiment. In Fig. 8, those parts that are common with the guide wire 210 of the third embodiment shown in Fig. 6 are given the same reference numerals. The guide wire 310 differs from the guide wire 210 in that it further includes an intermediate joint portion 44. The intermediate joint portion 44 is provided in a position between the distal end joint portion 40 and the proximal end joint portion 42 in the axial direction, is constituted by the same metal brazing as the distal end joint portion 40 and the proximal end joint portion 42, and fixes an intermediate portion of the outer coil 30 to the core shaft 220. The intermediate joint portion 44 is provided between the plated portion 224a and the plated portion 224b in the axial direction. Note that, in Figure 8, although the plated portion 224a and the magnetized portion 222a are provided further on the proximal end side than in Fig. 6, it is sufficient that the plated portion 224a and magnetized portion 222a are on the distal end side of the intermediate joint portion 44. For example, the plated portion 224a and the magnetized portion 222a may be formed so as to overlap the distal end joint portion 40 in the same manner as in Fig. 6.

With this configuration, as a result of the portion in which the diameter of the core shaft 220 is sectionally thicker and provided with the plated portions 224a and 224b, the flux that is applied to the joint portion prior to brazing in order to form the intermediate joint portion 44 can be more easily retained between the plated portion 224a and the plated portion 224b. Furthermore, when metal brazing is arranged in the region between the plated portion 224a and the plated portion 224b in order to form the intermediate joint portion 44, it is possible to suppress the molten metal brazing from flowing beyond the portion in which the core shaft 220 is sectionally thicker and provided with the plated portions 224a and 224b.

In the third embodiment and the modification of the third embodiment, although the two plated portions 224a and 224b are provided as high coercive force layers, the number of high coercive force layers may be one, or three or more. As a result of magnetizing the high coercive force layers together with the core shaft 220, it is possible to obtain the same effect of increasing the magnetic field strength of the magnetized portions compared to a case where a high coercive force layer is not provided. When a plurality of high coercive force layers are provided, the magnetized portions provided so as to correspond to the two or more high coercive force layers preferably have, as described in the second embodiment (not covered by the subject-matter of the claims), lines of magnetic of magnetic force extending in different directions from each other.

In the third embodiment, an external magnetic field is applied to each high coercive force layer to form a magnetized portion for each high coercive force layer, but other configurations are possible. For example, high coercive force layers may be formed over a wider area on the distal end portion of the core shaft 220, and a plurality of magnetized portions may be provided by individually applying an external magnetic field with respect to a plurality of locations that are separated in the axial direction in a region where consecutively provided high coercive force layers overlap. Similarly, in this case, it is preferable that the lines of magnetic force of at least two of the plurality of magnetized portions extend in different directions from each other.

### D. Fourth Embodiment:

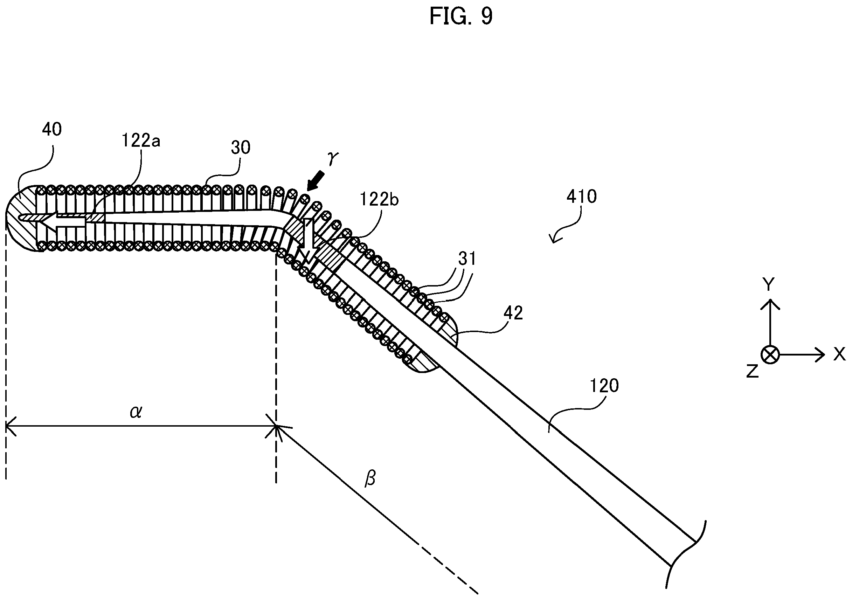

Fig. 9 is a partial sectional view showing, in the same manner as Fig. 1, a schematic configuration of the distal end portion of a guide wire 410 of a fourth embodiment. In Fig. 9, those parts that are common with the guide wire 110 of the second embodiment (not covered by the subject-matter of the claims) shown in Fig. 5 are given the same reference numerals. The guide wire 410 differs from the guide wire 110 in that the distal end portion of the guide wire 410 has a bent portion y that is bent in a specific direction. In Figure 9, the position of the bent portion y is indicated by the black arrow y. The bent portion y refers to a location where the axial direction, which extends in a fixed direction from the proximal end side, begins to bend. In the guide wire 410, the portion further on the distal end side than the bent portion y is referred to as a "pre-shaped portion α", and the portion further on the proximal side than the bent portion y is referred to as a "straight portion β". As a result of providing the pre-shaped portion α on the distal end portion of the guide wire, the operability of the distal end portion of the guide wire is improved, for example, and the blood vessel selectivity increases when the guide wire is used by being inserted in a blood vessel. In Fig. 9, although mutually perpendicular X, Y, and Z axes are illustrated in the same manner as in Fig. 1, in Fig. 9 the X-axis corresponds to the axial direction of the pre-shaped portion α of the guide wire 410, the Y-axis corresponds to the height direction of the pre-shaped portion α, and the Z-direction corresponds to the width direction of the pre-shaped portion α.

In the guide wire 410, the magnetized portion 122a is provided in the pre-shaped portion α, and the magnetized portion 122b is provided in the straight portion β. The magnetized portion 122a is also referred to as a "first magnetized portion", and the magnetized portion 122b is also referred to as a "second magnetized portion". In the present embodiment, the magnetized portion 122a is formed in a portion that includes the distal end of the core shaft 120. The magnetized portion 122a may not include the distal end of the core shaft 120, but as mentioned below, the position of the magnetized portion 122a is preferably as close to the distal end of the guide wire 410 as possible. In the present embodiment, the magnetized portion 122b formed in a position in which the end portion on the distal side of the magnetized portion 122b overlaps the bent portion y. The end portion on the distal end side of the magnetized portion 122b may be separated from the bent portion γ, but as mentioned below, the position of the magnetized portion 122b in the straight portion β is preferably as close to the bent portion y as possible.

Specifically, the distance from the bent portion y to the second magnetized portion 122b may be set to a distance that is provides sufficient accuracy when the second magnetized portion 122 is detected with a magnetic sensor to determine the orientation of the straight portion β and specify the position of the bent portion y. For example, the distance between the end portion on the distal end side of the magnetized portion 122b and the bent portion y is preferably shorter than the axial direction length of the pre-shaped portion α. Furthermore, the distance between the end portion on the distal end side of the magnetized portion 122b and the bent portion y is preferably shorter than the axial direction length of the magnetized portion 122b in the straight portion β. That is, the distance between the end portion on the distal end side of the magnetized portion 122b and the bent portion y is preferably shorter than at least one of the axial direction length of the pre-shaped portion α and the axial direction length of the magnetized portion 122b. In the fourth embodiment, although the axial direction of the guide wire 410 changes at the bent portion γ, even in this form, the magnetized portion 122a and the magnetized portion 122b are "separated from each other in the axial direction".

The measurement method of the distance from the bent portion y to the second magnetized portion 122b in the guide wire 410 will be described below. The distance from the bent portion y to the second magnetized portion 122b can, for example, be known from the pattern of the change in magnetic flux density obtained by measuring the magnetic flux density in the axial direction along the surface of the guide wire 410 using a magnetic sensor. Specifically, a location of the target guide wire that is magnetized is firstly specified. The location that is magnetized can be specified, for example, as a location where magnetic fine particles collect upon arranging the target guide wire on a sheet inside which magnetic fine particles have been sealed. Then, a magnetic sensor is used to measure the magnetic flux density in the axial direction along the surface of the guide wire over a wider area that includes the specified location that is magnetized.

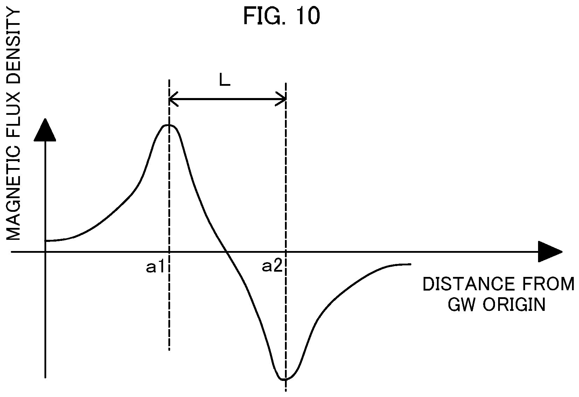

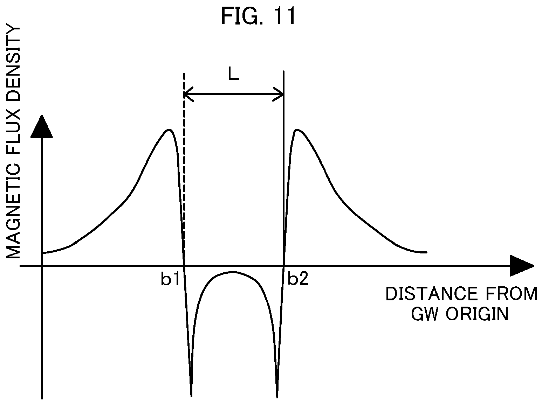

Fig. 10 is an explanatory diagram showing, as an example, a measurement of the change in magnetic flux density in the axial direction for a magnetized portion having a magnetization direction parallel to the axial direction. In Fig. 10 and Fig. 11, which is described below, the horizontal axis represents the distance toward the proximal end side from a predetermined origin, which is further on the distal end side of the straight portion β of the guide wire than the magnetized portion that is the measurement target, and the vertical axis represents the magnetic flux density. After specifying the location that is magnetized as described above, when the location that is magnetized is brought into contact with the magnetic sensor, and the direction of the magnetic flux density does not reverse when the guide wire is rotated about its axis on the magnetic sensor, it can be determined that the magnetization direction is parallel to the axial direction. When the magnetization direction is parallel to the axial direction, and the magnetic sensor is moved along the axial direction of the surface of the guide wire from the origin toward the proximal end side, as shown in Fig. 10, the magnetic flux density gradually increases and reaches a positive peak, decreases and reverses sign and reaches a negative peak, and then increases again. The positive peak position a1 is the end portion on the distal end side of the magnetized portion, and the negative peak position a2 is the end portion on the proximal end side of the magnetized portion. Further, the distance L between the position a1 and the position a2 is the axial direction length of the magnetized portion. When the magnetization direction of the magnetized portion is reversed, the sign of the magnetic flux density is reversed.

Fig. 11 is an explanatory diagram showing, as another example, a measurement of the change in magnetic flux density in a magnetized portion having a magnetization direction perpendicular to the axial direction in the same manner as Fig. 10. After specifying the location that is magnetized as described above, when the location that is magnetized is brought into contact with the magnetic sensor, and the direction of the magnetic flux density reverses when the guide wire is rotated about its axis on the magnetic sensor, it can be determined that the magnetization direction is perpendicular to the axial direction. When the magnetization direction is perpendicular to the axial direction, and the magnetic sensor is moved along the axial direction of the surface of the guide wire from the origin toward the proximal end side, as shown in Fig. 11, the magnetic flux density gradually increases and reaches a first positive peak, and then rapidly decreases and reverses sign and reaches a first negative peak. Then, the magnetic flux density increases close to zero before decreasing and reaching a second negative peak, rapidly increases and reverses sign and reaches a second positive peak, and then gradually decreases. The position b1 between the first positive peak and the first negative peak where the magnetic flux density becomes zero is the end portion on the distal end side of the magnetized portion, and the position b2 between the second negative peak and the second positive peak where the magnetic flux density becomes zero is the end portion on the proximal end side of the magnetized portion. Further, the distance L between the position b1 and the position b2 is the axial direction length of the magnetized portion.

As described above, from the pattern of change in magnetic flux density in the axial direction, which changes according to the magnetization direction, it is possible to specify the position of the end portion on the distal end side and the position of the end portion on the proximal end side in the axial direction of the magnetized portion, and specify the length of the magnetized portion in the axial direction as the distance between the end portion on the distal end side and the end portion on the proximal end side. The positional relationship between the bent portion y and the second magnetized portion 122b described above can be stated, in other words, as a region from the bent portion y to a position on the proximal end side having the axial direction length of the pre-shaped portion α, or a region from the bent portion y to a position on the proximal end side having the axial direction length of the second magnetized portion 122b overlapping with the second magnetized portion 122b. However, the second magnetized portion 122b may be more separated from the bent portion y on the proximal end portion side than the positional relationship above.

In Fig. 9, the direction of the external magnetic fields applied with respect to the to-be-magnetized member 21 for forming the magnetized portions 122a and 122b are indicated by white arrows. In the fourth embodiment, although the magnetized portion 122a and the magnetized portion 122b have different magnetization directions, they may be the same direction. However, as described in the second embodiment (not covered by the subject-matter of the claims), in order to suppress inconveniences due to the direction the lines of magnetic force of the magnetized portion 122a extend and the direction the lines of magnetic force of the magnetized portion 122b extend being parallel to each other, the angle formed between the two directions above is preferably 20° or more, more preferably 45° or more, and is preferably as close to 90° as possible. Here, the angle formed between the direction the lines of magnetic force of the magnetized portion 122a extend and the direction the lines of magnetic force of the magnetized portion 122b extend refers to the angle after the guide wire 410 is bent at the bent portion y.

For example, in order to produce the guide wire 410, the guide wire having the magnetized portions 122a and 122b is prepared in the same manner as in the second embodiment (not covered by the subject-matter of the claims), and the guide wire 410 is then obtained by forming the bent portion y by bending the prepared guide wire at a specific location and in a specific direction. Here, when magnetizing the predetermined locations of the to-be-magnetized member 21, or more specifically, the locations in which the magnetized portion 122a and the magnetized portion 122b are to be formed, the magnetization directions are set with consideration to the bending angle of the bent portion y that is subsequently formed, such that the angle formed between the direction the lines of magnetic force of the magnetized portion 122a extend and the direction the lines of magnetic force of the magnetized portion 122b extend forms the desired angle. The areas in which the magnetized portions 122a and 122b are formed are the areas of the to-be-magnetized member 21 that overlap with the air core coil 60 (see Fig. 3A), the permanent magnets 62 (see Fig. 4A) or the like described above in a direction perpendicular to the axial direction of the to-be-magnetized member 21.

Alternatively, when producing the guide wire 410, the magnetized portions 122a and 122b may be formed by performing the magnetizing step after forming the bent portion y. Furthermore, the guide wire 410 may be shipped in a state where the bent portion y is not formed, and the bent portion y may be formed as a result of an operator such as a physician bending the guide wire prior to use. In this case, in order to specify the location the bent portion y is to be formed, a marking which enables the position of the magnetized portion 122b to be externally recognized may be made on the surface of the guide wire. Furthermore, information that relates to the recommended bending angle may be provided together with the guide wire.

With this configuration, because the magnetized portions 122a and 122b are formed by magnetizing the core shaft 120 that is originally included in the guide wire 410, the same effects as in the first embodiment (not covered by the subject-matter of the claims) can be obtained. Furthermore, in the core shaft 120, because the plurality of magnetized portions 122a and 122b are provided separated from each other in the axial direction, the same effects as in the second embodiment (not covered by the subject-matter of the claims) are obtained. In addition, as a result of providing the bent portion γ, providing the magnetized portion 122a in the pre-shaped portion α, and providing the magnetized portion 122b in the straight portion β, the accuracy with which the three-dimensional movement of the distal end portion of the guide wire 410 is recognized can be further increased. This effect will be further described.

For example, when the guide wire 410 is inserted inside the body and is being used, and a load is applied to the distal end of the guide wire 410 and a deformation such as a bend occurs, the deformation mainly occurs further on the distal end side than the bent portion y. Furthermore, in the straight portion β, the location of the magnetized portion 122b is formed (specifically, the distance from the bent portion y to the magnetized portion 122b) and the magnetization direction of the magnetized portion 122b can be known in advance. Therefore, by detecting the strength and direction of the magnetic field of the magnetized portion 122b using a magnetic sensor, the axial direction of the straight portion β and the position of the bent portion y can be known. In the fourth embodiment, by using the position of the bent portion y and the axial direction of the straight portion β obtained as described above as a reference, and using the strength and direction of the magnetic field of the magnetized portion 122a detected by the magnetic sensor, it is possible to more accurately detect the three-dimensional movement of the distal end portion of the pre-shaped portion α provided with the magnetized portion 122a.

At this time, as described above, by providing the magnetized portion 122b in a position of the straight portion β which is closer to the bent portion γ, the accuracy with which the position of the bent portion y is specified by detecting the magnetized portion 122b using a magnetic sensor can be increased. As a result, the accuracy with which the state of the distal end portion of the guide wire 410 is detected can be further increased.

Further, by making the direction the lines of magnetic force of the magnetized portion 122a extend and the direction the lines of magnetic force of the magnetized portion 122b extend different from each other, the distance between the magnetized portions 122a and 122b can be made even shorter. When the directions the lines of magnetic force of the magnetized portions 122a and 122b extend are parallel to the axial direction and are in the same direction, it is necessary to ensure a certain distance between the magnetized portions 122a and 122b in order to make it easier to distinguish between the two using a magnetic sensor. When the directions the lines of magnetic force of the magnetized portions 122a and 122b extend are parallel to the axial direction and are in opposite directions, it is necessary to ensure a certain distance between the magnetized portions 122a and 122b in order to prevent the magnetic forces of the magnetized portions 122a and 122b from cancelling each other out. When the directions the lines of magnetic force of the magnetized portions 122a and 122b extend are different from the axial direction and are in the same direction, it is necessary to ensure a certain distance between the magnetized portions 122a and 122b in order to suppress the repulsion of the magnetic forces between the magnetized portion 122a and the magnetized portion 122b. When the directions the lines of magnetic force of the magnetized portions 122a and 122b extend are different from the axial direction and are in opposite directions, it is necessary to ensure a certain distance between the magnetized portions 122a and 122b in order to prevent the between the magnetized portion 122a and the magnetized portion 122b from forming a magnetic circuit. When the directions in which the lines of magnetic force of the magnetized portions 122a and 122b are different as in the fourth embodiment, the inconveniences described above are suppressed and the distance between the magnetized portions 122a and 122b can be made short. As a result, the pre-shaped portion α can be made even shorter. The length of the pre-shaped portion α may be subjected to limitations depending on the usage target of the guide wire (for example, the location inside the body the guide wire is to be inserted and used, such as the blood vessels of the heart or the blood vessels of the lower extremities). By shortening the length of the pre-shaped portion α, when using the guide wire 410, it is possible to suppress limitations on the application or the target, and it becomes possible to exhibit higher performance.

In the guide wire 410 of the fourth embodiment, as in the third embodiment, high coercive force layers are provided on the surface of the core shaft 120 in regions overlapping the portions in which the magnetized portions 122a and 122b are formed.

In the fourth embodiment, the two magnetized portions 122a and 122b are provided, but three or more magnetized portions may be provided. In this case, in addition to the magnetized portion 122a provided on the distal end of the pre-shaped portion α and the magnetized portion 122b provided in the straight portion β, by providing another magnetized portion on the proximal end side of the magnetized portion 122a in the pre-shaped portion α, the accuracy with which the state of the readily deformable pre-shaped portion α is detected can be increased.

### E. Fifth Embodiment:

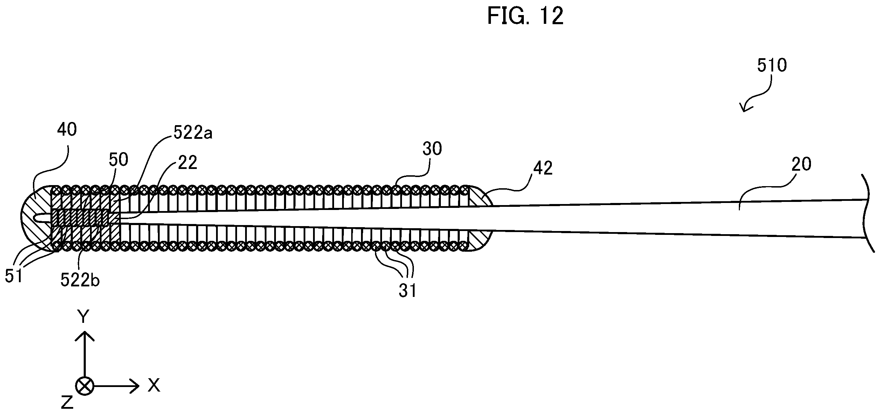

Fig. 12 is a partial sectional view showing, in the same manner as Fig. 1, a schematic configuration of the distal end portion of a guide wire 510 of a fifth embodiment. In the fifth embodiment, those parts that are common with the guide wire 10 of the first embodiment (not covered by the subject-matter of the claims) are given the same reference numerals. The guide wire 510 differs from the guide wire 10 in that, in addition to further including an inner coil 50, a magnetized portion 522a formed in the outer coil 30 and a magnetized portion 522b formed in the inner coil 50 are provided in addition to the magnetized portion 22 formed in the core shaft 20. In the fifth embodiment, in addition to the core shaft 20, the outer coil 30 and the inner coil 50 correspond to the "magnetized member". In Fig. 12, the inner coil 50 is shown as an exterior shape rather than as a cross-section.

The inner coil 50 is a substantially cylindrical member arranged inside the outer coil 30 such that the core shaft 20 penetrates through the interior. In the present embodiment, although the inner coil 50 is a single-thread coil formed by winding a single wire 51, it may also be a multi-thread coil formed by winding a plurality of wires 51 in a multi-thread pattern, a single-thread strand coil formed by winding a single strand formed by twisting together a plurality of wires 51, or a multi-thread strand coil formed using a plurality of strands formed by twisting together a plurality of wires 51 and winding the strands in a multi-thread pattern. Furthermore, the inner coil 50 may be configured by an arbitrary combination of single-thread coils, multi-thread coils, single-thread strand coils, and multi-thread strand coils. The wire 51 constituting the inner coil 50 is formed thinner than the wire 31 constituting the outer coil 30, and the axial direction length of the inner coil 50 is formed shorter than the axial direction length of the outer coil 30.

The wire 51 constituting the inner coil 50 of the present embodiment is formed of stainless steel, and has an austenite phase and a martensite phase as phases with different crystal structures. The inner coil 50 is formed by shaping a wire 51 composed of stainless steel that undergoes processing-induced martensitic transformation into a coil shape, and then causing a martensitic transformation to occur in at least a portion of the metal structure constituting the wire 51. The magnetized portion 522b is formed by applying an external magnetic field with respect to such an inner coil 50.

Examples of the stainless steel constituting the wire 51 that undergoes a processing-induced martensitic transformation include SUS304, SUS316 and SUS302, which are austenitic stainless steels, SUS444 and SUS434, which are ferritic stainless steels, and SUS630, which is a precipitation hardening stainless steel.

In the fifth embodiment, like the wire 51, the wire 31 constituting the outer coil 30 is formed of a stainless steel that undergoes a processing-induced martensitic transformation, and a martensitic transformation occurs in at least a portion of the metal structure constituting the wire 31.

When assembling the guide wire 510, the distal end portion of the core shaft 20 is inserted into the inner coil 50, and the inner coil 50 and the core shaft 20 are also accommodated inside the outer coil 30. Then, the distal end of the core shaft 20 and the distal ends of the outer coil 30 and inner coil 50 are brazed together to form the distal end joint portion 40. Furthermore, the proximal end of the outer coil 30 and the core shaft 20 are brazed together to form the proximal end joint portion 42, which completes the guide wire 510. Then, an external magnetic field is applied with respect to the assembled guide wire 510, which forms the magnetized portion 22 in the core shaft 20, forms the magnetized portion 522a in the outer coil 30, and forms the magnetized portion 522b in the inner coil 50, and completes the guide wire 510. The application of the external magnetic field can, for example, be performed in the same manner as the method described in Fig. 3A or Fig. 4A, and the direction of the applied magnetic field can be arbitrarily set. As a result, the magnetized portions 22, 522a and 522b can be formed in the same axial direction position of the core shaft 20, the outer coil 30, and the inner coil 50.

With this configuration, because the magnetized portion 22 is formed by magnetizing the core shaft 20 that is originally included in the guide wire 510, the same effects as in the first embodiment (not covered by the subject-matter of the claims) can be obtained. Further, because the magnetized portion 522a of the outer coil 30 and the magnetized portion 522b of the inner coil 50 are provided in addition to the magnetized portion 22, the magnetic field strength of the distal end portion of the guide wire 510 increases, and the accuracy with which the position of the distal end portion of the guide wire 510 is detected can be increased.

In Fig. 12, the magnetized portions 22, 522a and 522b are each formed in the core shaft 20, the outer coil 30, and the inner coil 50. In contrast, one or more of the core shaft 20, the outer coil 30, and the inner coil 50 may be formed of a different material from the ferromagnetic material, and the magnetized portions may be provided in only the other members that contain martensitic stainless steel, which is a ferromagnetic material. For example, the core shaft 20 may be formed of a different material from the ferromagnetic material, and the magnetized portions may be provided in only the outer coil 30 and the inner coil 50. Alternatively, it is possible to provide the magnetized portion 22 in the core shaft 20, or provide a magnetized portion in only one of the outer coil 30 and the inner coil 50 without providing a magnetized portion 22 in the core shaft 20. If a magnetized portion containing magnetized martensitic stainless steel is formed in the distal end portion of the guide wire, an increase in size and rigidity of the guide wire can be suppressed, and the position of the distal end portion of the guide wire can be detected.

Furthermore, like the first embodiment (not covered by the subject-matter of the claims), it is possible for the inner coil 50 to not be provided, and the magnetized portions 22 and 522a to be formed in the core shaft 20 and the outer coil 30. Alternatively, an external magnetic field may be applied with respect to only the to-be-magnetized member 21, which forms the core shaft 20, and the guide wire 510 may be assembled using the core shaft 20 formed with the magnetized portion 22. In this case, the outer coil 30 and inner coil 50 can be magnetized by the magnetic force of the magnetized portion 22 formed in the core shaft 20.

Moreover, the magnetized portion 22 is provided in the core shaft 20, as in the third embodiment, a high coercive force layer is provided on the core shaft 20 in a region overlapping the portion in which the magnetized portion 22 is formed.

In the fifth embodiment, although the magnetized portions 22, 522a and 522b are provided in one location in the axial direction, the magnetized portions may be provided in the core shaft 20, the outer coil 30 and the inner coil 50 in a plurality of locations in the axial direction. The magnetized portions that are provided in the same position in the axial direction can be formed at the same time as magnetized portions having the same magnetization direction. When the magnetized portions are provided in a plurality of locations in the axial direction, it is preferable that the magnetization directions are different in at least two locations, and that the lines of magnetic force of the magnetized portions that are formed extend are in different directions from each other.

Furthermore, when the magnetized portions are provided in a plurality of locations in the axial direction and in a plurality of members such as the core shaft 20 and the outer coil 30, a bent portion y may be provided in the guide wire as in the fourth embodiment. In this case, one of the plurality of locations mentioned above may be provided further on the distal end side than the bent portion γ, and another one of the locations may be provided further on the proximal end side than the bent portion y.

### F. Sixth Embodiment:

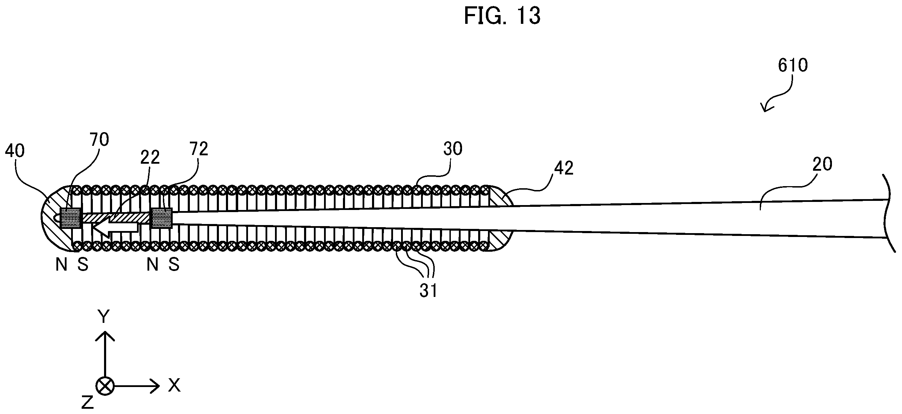

Fig. 13 is a partial sectional view showing, in the same manner as Fig. 1, a schematic configuration of the distal end portion of a guide wire 610 of a sixth embodiment. In the sixth embodiment, those parts that are common with the guide wire 10 of the first embodiment (not covered by the subject-matter of the claims) are given the same reference numerals. The guide wire 610 differs from the guide wire 10 in that it further includes the magnets 70 and 72.

The magnets 70 and 72 are permanent magnets arranged on both sides of the magnetized portion 22 so as to sandwich the magnetized portion 22 along the axial direction, and are fixed to the core shaft 20. The directions the lines of magnetic force of the magnets 70 and 72 extend are the same as that of the magnetized portion 22. In Fig. 13, as an example, the direction of the external magnetic field applied to the to-be-magnetized member 21, which forms the core shaft 20, in order to form the magnetized portion 22 is indicated by the white arrow, and the direction of the magnetic poles of the magnets 70 and 72 are also shown. Examples of permanent magnets constituting the magnets 70 and 72 that can be adopted include various known permanent magnets such as neodymium magnets, samarium cobalt magnets, ferrite magnets, and alnico magnets. Various modes can be used for the configuration when arranging the magnets 70 and 72. For example, the magnets 70 and 72 may be formed in a ring shape, and the core shaft 20 may then be fitted into the ring holes. Alternatively, the magnets 70 and 72 may be placed in contact with the surface of the core shaft 20 and adhered to the surface of the core shaft 20.

With this configuration, because the magnets 70 and 72 are arranged that sandwich the magnetized portion 22 and have lines of magnetic force that extend in the same direction as the magnetized portion 22, it is possible to suppress a decrease in the magnetic force of the magnetized portion 22. In the present embodiment, because the magnets 70 and 72 only need to suppress the demagnetization of the magnetized portion 22 and do not need to contribute to the detection of the position of the distal end portion of the guide wire 610, the size can be further reduced. In particular, in the present embodiment, as shown in Fig. 13, because the magnet 70 is arranged so as to be embedded inside the distal end joint portion 40, it is possible to suppress an increase in the size and rigidity of the guide wire 610 due to providing the magnet 70. The magnet 70 may be arranged outside the distal end joint portion 40 as long as the increase in the size and rigidity of the guide wire 610 is within an allowable range. Although the magnets 70 and 72 do not have to be in contact with the magnetized portion 22, it is preferable that the distance between the magnets 70 and 72 and the magnetized portion 22 is small in order to increase the effect of suppressing the demagnetization of the magnetized portion 22.

Furthermore, as in the third embodiment, a high coercive force layer is provided on the surface of the core shaft 20 in a region overlapping the portion in which the magnetized portion 22 is formed. Alternatively, like the fifth embodiment, the magnetized portion 522a may also be formed in the outer coil 30 using the outer coil 30 containing martensitic stainless steel. Furthermore, like the fifth embodiment, the magnetized portion 522b may also be provided in the inner coil 50 using the inner coil 50 containing martensitic stainless steel.

In the sixth embodiment, although a single magnetized portion 22 is provided in the core shaft 20, a plurality of magnetized portions that are separated from each other in the axial direction may be provided. Here, like the fourth embodiment, a bent portion y may be provided in the guide wire, at least one magnetized portion among the plurality of magnetized portions described above may be provided further on the distal end side than the bent portion γ, and one of the other magnetized portions may be provided further on the proximal end side than the bent portion y. In the core shaft 20, when the magnetized portions are provided in a plurality of locations in the axial direction, it is preferable that the magnetization directions are different in at least two locations, and the lines of magnetic force of the magnetized portions that are formed extend in different directions from each other. When the magnetized portions are provided in a plurality of locations in the axial direction, the magnets 70 and 72 fixed to the core shaft may be arranged so as to sandwich a magnetized portion having lines of magnetic force extending in a direction parallel to the axial direction.

### G. Other Embodiments:

(G1) In the embodiments described above, although the member to be processed is formed of a stainless steel that undergoes a processing-induced martensitic transformation, and the magnetized portion is formed by applying an external magnetic field with respect to the to-be-magnetized member in which a martensitic transformation has occurred due to processing, other configurations are also possible. For example, the entire member for forming magnetized portion, such as the core shaft, the outer coil, and the inner coil may be prepared using a ferromagnetic stainless steel such as martensitic stainless steel (such as SUS410) or ferritic stainless steel (such as SUS430).

(G2) In the embodiments described above, the medical device in which the magnetized portion for position detection is provided is a guide wire, but other configurations are also possible. In addition to guide wires, the medical device includes catheters and stylettes. A medical device formed in an elongated outer shape and having a distal end inserted inside the body may have a magnetized portion containing magnetized martensitic stainless steel in the distal end portion.

The present disclosure can be implemented in various configurations without departing from the scope of the present disclosure.

### DESCRIPTION OF REFERENCE NUMERALS

10, 110, 210, 310, 410, 510, 610 Guide wire / Medical device
20, 120, 220 Core shaft
21 To-be-magnetized member
22, 122a, 122b, 222a, 222b, 522a, 522b Magnetized portion
30 Outer coil
31 Wire
40 Distal end joint portion
42 Proximal end joint portion
44 Intermediate joint portion
50 Inner coil
51 Wire
60 Air core coil
62 Permanent magnet
70, 72 Magnet
224a, 224b Plated portion / High coercive force layer γ Bent portion.

## Claims

1. A medical device (210; 310; 410; 510; 610) formed in an elongated outer shape and having a distal end side inserted inside a body when in use, comprising:
a magnetized member in which a magnetized portion (22; 122a; 122b; 222a; 222b; 522a; 522b) containing magnetized martensitic stainless steel is formed in a portion of the distal end side; **characterized in that**
the medical device (210; 310; 410; 510; 610) further comprises
a high coercive force layer (224a; 224b) formed so as to cover at least a portion of a surface of the magnetized member, and contains a material with a higher coercive force than martensitic stainless steel, wherein the magnetized portion (22, 122a; 122b; 222a; 222b; 522a; 522b) has the high coercive force layer (224a; 224b) magnetized together with the magnetized member and/or
a pair of permanent magnets (70; 72) having lines of magnetic force extending in the same direction as the magnetized portion (22;) is provided on both sides of the magnetized portion (22).

2. The medical device (210; 310; 410; 510; 610) according to claim 1, wherein
the magnetized member is a core shaft (20; 120; 220), and
the magnetized portion (22; 122a; 122b; 222a; 222b; 522a; 522b) is provided in a distal end portion (40) of the core shaft.

3. The medical device (210; 310; 410; 610) according to claim 1 or 2, wherein
a plurality of the magnetized portions (22; 122a; 122b; 222a; 222b) are provided spaced apart from each other in an axial direction of the medical device (210; 310; 410; 610).

4. The medical device (210; 310; 410; 610) according to claim 3, wherein
at least two magnetized portions (22; 122a; 122b; 222a; 222b) among the plurality of magnetized portions (22; 122a; 122b; 222a; 222b) have lines of magnetic force extending in different directions from each other.

5. The medical device (410; 610) according to claim 3, wherein
a distal end portion (40) of the medical device (410) is provided with a bent portion (γ) at which the medical device (410) is bent in a specific direction,
a first magnetized portion (22; 122a), being one of the plurality of magnetized portions (22; 122a; 122b), is provided further toward a distal end side than the bent portion (γ), and
a second magnetized portion (22; 122b), being another one of the plurality of magnetized portions (22; 122a; 122b), is provided further toward a proximal end side than the bent portion (γ).

6. The medical device (410; 610) according to claim 5, wherein
the first magnetized portion (22, 122a) and the second magnetized portion (22, 122b) have lines of magnetic force extending in different directions from each other.

## Patentansprüche

1. Medizinisches Gerät (210; 310; 410; 510; 610) mit einer länglichen Außenform und einer distalen Endseite, die bei Gebrauch in einen Körper eingeführt ist, aufweisend:
ein magnetisiertes Teil, in dem ein magnetisierter Abschnitt (22; 122a; 122b; 222a; 222b; 522a; 522b), der magnetisierten martensitischen Edelstahl enthält, in einem Abschnitt der distalen Endseite ausgebildet ist;
**dadurch gekennzeichnet, dass**
das medizinische Gerät (210; 310; 410; 510; 610) ferner
eine Schicht (224a; 224b) mit hoher Koerzitivkraft aufweist, die so gebildet ist, dass sie mindestens einen Abschnitt einer Oberfläche des magnetisierten Teils bedeckt, und ein Material mit einer höheren Koerzitivkraft als martensitischer Edelstahl enthält, wobei der magnetisierte Abschnitt (22, 122a; 122b; 222a; 222b; 522a; 522b) die Schicht (224a; 224b) mit hoher Koerzitivkraft zusammen mit dem magnetisierten Teil magnetisiert aufweist, und/oder
ein Paar Permanentmagnete (70; 72) mit Magnetkraftlinien, die sich in derselben Richtung wie der magnetisierte Abschnitt (22;) erstrecken, auf beiden Seiten des magnetisierten Abschnitts (22) vorgesehen ist.

2. Medizinisches Gerät (210; 310; 410; 510; 610) gemäß Anspruch 1, wobei
das magnetisierte Teil ein Kernschaft (20; 120; 220) ist, und
der magnetisierte Abschnitt (22; 122a; 122b; 222a; 222b; 522a; 522b) in einem distalen Endabschnitt (40) des Kernschafts vorgesehen ist.

3. Medizinisches Gerät (210; 310; 410; 610) gemäß Anspruch 1 oder 2, wobei
eine Vielzahl der magnetisierten Abschnitte (22; 122a; 122b; 222a; 222b) in axialer Richtung des medizinischen Geräts (210; 310; 410; 610) voneinander beabstandet vorgesehen sind.

4. Medizinisches Gerät (210; 310; 410; 610) gemäß Anspruch 3, wobei
mindestens zwei magnetisierte Abschnitte (22; 122a; 122b; 222a; 222b) unter der Vielzahl von den magnetisierten Abschnitten (22; 122a; 122b; 222a; 222b) Magnetkraftlinien aufweisen, die sich in unterschiedlichen Richtungen voneinander erstrecken.

5. Medizinisches Gerät (410; 610) gemäß Anspruch 3, wobei
ein distaler Endabschnitt (40) des medizinischen Geräts (410) mit einem gebogenen Abschnitt (γ) vorgesehen ist, an dem das medizinische Gerät (410) in eine bestimmte Richtung gebogen ist,
ein erster magnetisierter Abschnitt (22; 122a), der einer der Vielzahl von den magnetisierten Abschnitten (22; 122a; 122b) ist, weiter in Richtung einer distalen Endseite als der gebogene Abschnitt (γ) vorgesehen ist, und
ein zweiter magnetisierter Abschnitt (22; 122b), der ein weiterer der Vielzahl von den magnetisierten Abschnitten (22; 122a; 122b) ist, weiter in Richtung einer proximalen Endseite als der gebogene Abschnitt (γ) vorgesehen ist.

6. Medizinisches Gerät (410; 610) nach Anspruch 5, wobei
der erste magnetisierte Abschnitt (22, 122a) und der zweite magnetisierte Abschnitt (22, 122b) Magnetkraftlinien aufweisen, die sich in voneinander unterschiedlichen Richtungen erstrecken.

## Revendications

1. Dispositif médical (210 ; 310 ; 410 ; 510 ; 610) formé selon une forme extérieure allongée et présentant une extrémité distale qui est insérée à l'intérieur d'un corps lors de l'utilisation, comprenant :
un élément magnétisé dans lequel une partie magnétisée (22 ; 122a ; 122b ; 222a ; 222b ; 522a ; 522b) contenant de l'acier inoxydable martensitique magnétisé est formée dans une partie du côté de l'extrémité distale ;
**caractérisé en ce que**
le dispositif médical (210 ; 310 ; 410 ; 510 ; 610) comprend en outre
une couche à force coercitive élevée (224a ; 224b) formée de manière à recouvrir au moins une partie d'une surface de l'élément magnétisé, et contenant un matériau avec une force coercitive supérieure à celle de l'acier inoxydable martensitique, dans laquelle la partie magnétisée (22, 122a ; 122b ; 222a ; 222b ; 522a ; 522b) présente la couche à force coercitive élevée (224a ; 224b) magnétisée conjointement avec l'élément magnétisé et/ou
une paire d'aimants permanents (70 ; 72) présentant des lignes de force magnétique s'étendant dans la même direction que la partie magnétisée (22) est prévue des deux côtés de la partie magnétisée (22).

2. Dispositif médical (210 ; 310 ; 410 ; 510 ; 610) selon la revendication 1, dans lequel
l'élément magnétisé est un arbre central (20 ; 120 ; 220), et
la partie magnétisée (22 ; 122a ; 122b ; 222a ; 222b ; 522a ; 522b) est prévue dans une partie d'extrémité distale (40) de l'arbre central.

3. Dispositif médical (210 ; 310 ; 410 ; 610) selon la revendication 1 ou 2, dans lequel
une pluralité de parties magnétisées (22 ; 122a ; 122b ; 222a ; 222b) sont disposées espacées les unes des autres dans une direction axiale du dispositif médical (210 ; 310 ; 410 ; 610).

4. Dispositif médical (210 ; 310 ; 410 ; 610) selon la revendication 3, dans lequel
au moins deux parties magnétisées (22 ; 122a ; 122b ; 222a ; 222b) parmi la pluralité de parties magnétisées (22 ; 122a ; 122b ; 222a ; 222b) présentent des lignes de force magnétique s'étendant dans des directions différentes l'une de l'autre.

5. Dispositif médical (410 ; 610) selon la revendication 3, dans lequel
une partie distale (40) du dispositif médical (410) est munie d'une partie courbée (γ) au niveau de laquelle le dispositif médical (410) est courbé dans une direction spécifique,
une première partie magnétisée (22 ; 122a), faisant partie de la pluralité de parties magnétisées (22 ; 122a ; 122b), est située plus près d'une extrémité distale que la partie courbée (γ), et
une seconde partie magnétisée (22 ; 122b), étant une autre de la pluralité de parties magnétisées (22 ; 122a ; 122b), est prévue plus près d'une extrémité proximale que la partie courbée (γ).

6. Dispositif médical (410 ; 610) selon la revendication 5, dans lequel
la première partie magnétisée (22, 122a) et la seconde partie magnétisée (22, 122b) présentent des lignes de force magnétique s'étendant dans des directions différentes l'une de l'autre.
